# EUROPÄISCHE PATENTSCHRIFT

(11) **EP 0 684 299 B1**
(45) Veröffentlichungstag und Bekanntmachung des Hinweises auf die Patenterteilung: **22.11.2000**
(21) Anmeldenummer: 95107285.9
(22) Anmeldetag: 13.05.1995
(51) Int. Cl.: C11B 9/00, A23L 1/236, A23L 1/226

(54) **Dihydrofarnesal**
Dihydrofarnesal
Dihydrofarnesal

(30) Priorität: 26.05.1994 CH 163394
(43) Veröffentlichungstag der Anmeldung: 29.11.1995
(73) Patentinhaber: Givaudan SA, 1214 Vernier-Genève (CH)
(72) Erfinder: Kaiser, Roman, CH-8610 Uster (CH)
(74) Vertreter: Buntz, Gerhard

(56) Entgegenhaltungen:
- DE-A- 2 719 735
- JOURNAL OF THE SCIENCE OF FOOD AND AGRICULTURE, Bd. 36, 1985, BARKING GB, Seiten 1145-1154, XP002025169 BAALIOUAMER A. ET AL.: "Qualitative and quantitative analysis of Petitgrain Eureka Lemon essential oil by fused silica capillary column gas chromatography mass spectrometry"
- S.ARCTANDER: "Perfume and flavor chemicals" 1969 , S.ARCTANDER , MONTCLAIR,N.J. XP002025170 * 1378:FARNESAL * * 2897:TETRAHYDRO FARNESAL *

## Beschreibung

Die Erfindung betrifft die Verwendung von 2(3)-Dihydrofarnesal 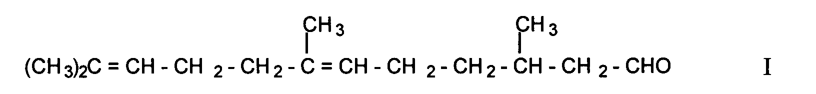 als Riech- und/oder Geschmacksstoff und 2(3)-Dihydrofarnesale enthaltende Riech-und/oder Geschmacksstoffkompositionen.

Die Formel I soll sämtliche geometrischen Isomeren dieses Terpenderivats umfassen, also sowohl die 6 E- als auch 6 Z-Form, also insbesondere die Isomeren der Formeln 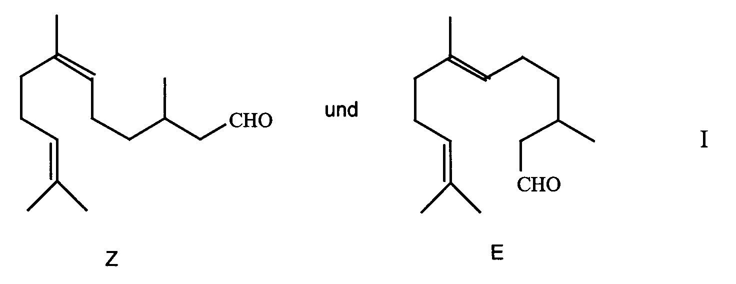 und - obschon üblicherweise als Racemat vorliegend - auch die beiden, durch das Asymmetriezentrum in Stellung 3 auftretenden optischen Antipoden.

Die Verbindung I ist geruchlich äusserst intressant, und die Erfindung betrifft demgemäss auch die Verwendung von I als Riech- und/oder Geschmackstoff.

Die Verbindung I ist bekannt (A. Baalionamer et al., J.. Sci. Food Agric. 1985, 36, 1145-1154) und kann in der dem Fachmann bekannten Weise, beispielsweise durch Hydrierung von Farnesal hergestellt werden. Auch die Herstellung der einzelnen Isomeren kann in an sich bekannter Weise erfolgen, also z.B. aus Z- oder E-Geranylaceton.

Die erfindungsgemäss als Riech- und/oder Geschmackstoff verwendete Verbindung der Formel I zeichnet sich durch angenehme, blumige Noten aus.

Das bevorzugtere 6-(E)-2(3)-Dihydrofarnesal zeigt einen frisch-blumigen, aldehydischen, an Maiglöckchen und auch andere Blumen erinnernden Duft, der leicht mit frischem Wasser, Ozon, Meer etc. assoziiert werden kann. Die für das E-Isomere charakteristischen Duftaspekte werden auch in den Blütendüften gewisser Lilienarten, Citrusarten, Orchideenarten, Vertretern der Familie der Apocynaceae, Vertretern der Familie der Asclepiadaceae und Kakteenarten angetroffen.

Aehnliche olfaktorische Eigenschaften zeigt das entsprechende 6-(Z)-Isomere, wobei allerdings hier die Intensität etwas geringer ist, und zudem der Gesamtgeruch etwas weniger erfrischend wirkt. Dementsprechend können an Stelle des reinen (E)-2(3)-Dihydrofarnesals auch die synthetisch bequemer zugänglichen Gemische der Z- und E-Formen verwendet werden.

Auf Grund dieser olfaktorischen Eigenschaften eignet sich I - als Gemisch, oder als E- oder Z-form - zur Verwendung bei der Kreation der verschiedenartigsten Parfüm-Typen. Ein besonders wichtiges Anwendungsgebiet stellen blumige Kompositionen dar, welche durch Zusatz von I eine angenehme, sehr natürlich wirkende Frische erhalten und nun zudem viel abgerundeter wirken. In diesem Zusammenhang wurde insbesondere festgestellt, dass Parfümerieprodukte mit charakteristischen Duftnoten, deren harmonisches Einbauen in eine Komposition oft Schwierigkeiten bereitet, bei Mitverwendung von 2(3)-Dihydrofarnesal einfacher hand-zuhaben sind und zudem in höherer Konzentration verwendet werden können.

Die 2(3)-Dihydrofarnesale I zeigen aber auch sehr interessante und wertvolle Eigenschaften in Kompositionen, die durch holzige, hesperidenartige, grün-krautige, grün-fruchtige, fruchtige, würzige, ambraartige und artige, grün-krautige, grün-fruchtige, fruchtige, würzige, ambraartige und moschusartige Noten geprägt sind. Ganz allgemein kann gesagt werden, dass die Verbindung I ein sehr hohes Integrationsvermögen in ParfümKompositionen besitzt und daher über einen weiten Konzentrationsbereich eingesetzt werden können. Im allgemeinen bewegt sich aber die Anwendungskonzentration zwischen ca. 0,1 und ca. 40%, vorzugsweise zwischen ca. 3 und ca. 20%.

Die erfindungsgemäss als Riech- und/oder Geschmackstoff verwendete Verbindung der Formel I zeichnet sich also, wie erwähnt, durch angenehme Noten aus. Die Verbindung I verbindet sich mit zahlreichen bekannten Riechstoffingredienzien natürlichen oder synthetischen Ursprungs, wobei die Palette der natürlichen Rohstoffe sowohl leichtals auch mittel- und schwerflüchtige Komponenten, und diejenige der Synthetika Vertreter aus praktisch allen Stoffklassen umfassen kann, wie dies aus der folgenden Zusammenstellung ersichtlich ist:
- Naturprodukte: Basilikumöl, Baummoos Absolue, Beifussöl, Bergamotteöl, Cassiaöl, Cassisknospen Absolue, Zedernholzöl, Ciste Labdanum, Civette, Corianderöl, Eichenmoos, Elemiöl, Fichtennadelöl, Galbanum, Geraniumöl, Gewürznelkenöl, Jasmin Absolue und sein synthetischer Ersatz, Jonquille Absolue, Kamillenöl, Labdanum, Lavendelöl, Mandarinenöl, Mastix Absolue, Mentha citrata-öl, Myrrhenöl, Palmarosaöl, Patchouliöl, Petitgrainöl Paraguay, Rosenöl, Sandelholzöl, Thymianöl, Vassouraöl, Styrax, Birkenteer, Vetiveröl, Weihrauch, Ylang-Ylang-Oel, Zitronenöl, Zimtrindenöl.
- Alkohole: Citronellöl, Dimetol® (2,6-Dimethyl-heptan-2-ol), Geraniol, cis-3-Hexanol, Linalool, Nonadyl® (6,8-Dimethyl-nonan-2-ol), Phenyläthylalkohol, Rhodinol, Sandela® (3-Isocamphyl-5-cyclohexanol), Sandalore® (3-Methyl-5 (2',2',3'-trimethyl-cyclopenta-3'-en-1'-yl)-pentan-2-ol), Terpineol, etc.
- Aldehyde: α-Amylzimtaldehyd, Cyclamenaldehyd, Decanal, Phenylacetaldehyd, Dodecanal, Heliotropin, α-Hexylzimtaldehyd, Hydroxycitronellal, Lyral, Adoxal® (2,6,10-Trimethyl-9-en-1-al), Undecanal, ω-Undecylenaldehyd, Anisaldehyd, Vanillin, etc.
- Ketone: Isoraldeine® (Isomethyl-α-jonon), α-Jonon, β-Jonon, 3-Phenylisocaranon, Vertofix® (=acetyliertes Cedernholzöl), p-Methylacetophenon, β-Damascon, Geranylaceton, Muscon, Dihydro-β-ionon, etc.
- Ester: Acetessigsäureäthylester, Amylsalicylat, Benzylacetat, Cedrylacetat, Cinnamylformiat, Citronellylacetat, Geranylacetat, cis-3-Hexenylacetat, cis-3-Hexanylbenzoat, Linalylacetat, Linalylantharnilat, Methyldihydrojasmonat, Methambrat® (1-Acetoxy-1-methyl-2-sec.-butylcyclohexan), Myraldylacetat® (4-(4-Methyl-3-pentenyl)-cyclohex-3-en-1-yl-carbinylacetat), Phenoxyäthylisobutyrat, Phenyläthyltiglat, cis-3-Hexenylsalicylat, Styrallylacetat, Terpenylacetat, 2,3,6,6-Tetramethylcyclohexy-2-en-carbonsäure-äthylester, Vetivenylacetat, ortho-tert.-Butylcyclohexyl-acetat, etc.
- Verschiedene: Cumarin, Epoxycedren, Eugenol, Fixolide® (1,1,2,4,4,7-Hexamethyl-6-acetyl-1,2,3,4-tetrahydronaphthalin), Galaxolid® (1,3,4,6,7,8-Hexahydro-4,6,6,7,8,8-hexamethyl-cyclopentyl-γ-2-benzopyran), Heliotropin, Indol, Indolen, Isoeugenol, Isobutylchinolin, Jasmonyl (1,3-Diacetoxy-nonan), Limonen, p-Menthan-8-thiol-3-on, Madrox® (1-Methylcyclododecyl-methyläther), Methyleugenol, Musk 174® (12-Oxahexadecanolid), γ-Nonalacton, α-Undecalacton, ω-Pentadecanolid, Schiffsche Basen, z.B. von Methylanthranilat und der in der Parfümerie üblichen Aldehyde, etc.

Die Verbindung der Formel I lässt sich in weiten Grenzen einsetzen, die bespielsweise von 0.1 (Detergentien) - 40 Gew.-% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 3 und 20%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, etc.).

Die Verbindung I kann demgemäss bei der Herstellung von Kompositionen und - wie obige Zusammenstellung zeigt - unter Verwendung einer breiten Palette bekannter Riechstoffe bzw. Riechstoffgemische verwendet werden. Bei der Herstellung solcher Kompositionen können die oben angeführten bekannten Riechstoffe bzw. Riechstoffgemische nach (dem Parfümeur bekannter) Art und Weise verwendet werden, wie z.B. aus W.A. Poucher, Perfumes, Cosmetics, Soaps 2, 7. Auflage, Chapman und Hall, London 1974 hervorgehend.

Die Verbindung der Formel I ist ebenfalls vorzüglich geeignet zur Verwendung in Aromen der verschiedensten Art.

Als Geschmackstoff kann die Verbindung I beispielsweise zur Abrundung, Verbesserung, Verstärkung, Steigerung des Frischeeffektes, Modifizierung von (Citrus-) Fruchtaromen, z.B. Aprikosen-, Pfirsich-Mango-, Guava-, Passionsfrucht-, Ananas-, Bananenaromen verwendet werden. Aber auch Kräuteraromen kommen in Frage. Als Anwendungsgebiet dieser Aromen kommen beispielsweise Nahrungsmittel (Joghurt, Süsswaren etc.), Genussmittel (Tee, etc.) und Getränke (Limonade etc.) in Frage.

Die ausgeprägten geschmacklichen Qualitäten der Verbindung I (frische Blumennote mit aldehydischem Aspekt, die sich gut mit den verschiedenartigsten Fruchtnoten kombinieren lässt) ermöglichen die Verwendung als Aromastoff in geringen Konzentrationen. Eine geeignete Dosierung umfasst beispielsweise den Bereich von 0.1 ppm - 100 ppm, vorzugsweise den Bereich von 0.5 ppm - 50 ppm im Fertigprodukt d.h. dem aromatisierten Nahrungsmittel, Genussmittel oder Getränk.

Bei der Aromatisierung von beispielsweise Mundpflegemitteln kann die Dosierung jedoch auch höher liegen und einen grösseren Bereich umfassen, beispielsweise den Bereich von 1 bis 1000 ppm, vorzugsweise 30 - 100 ppm.

Die Verbindung kann auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0.1-10, insbesondere 0.5-3 Gew. %. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wie z.B. J. Merory, Food Flavorings, Composition, Manufacture and Use, Second Edition. The Avi Publishing Company, Inc., Westport, Conn. 1968, oder G. Fenaroli, Fenroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredienzien, etc. in Frage:

Gummi arabicum, Tragant, Salze oder Brauereihefe, Alginate, Carrageen oder ähnliche Absorbenzien; Indole, Maltol, Dienale, Gewürzollleoresine, Raucharomen; Gewürznelken, Diacetyl, Natriumcitrat; Mononatriumglutamat, Dinatriuminosin-5'-monophosphat (IMP), Dinatriumguenosin-5-phosphat (GMP); oder spezielle Aromastoffe, Wasser, Aethanol, Prypylenglykol, Glycerin, etc.

### Beispiel 1

### Herstellung von Dihydrofarnesal I

110,0 g (0,50 Mol) Farnesal (Gemisch der 4 Isomeren), gelöst in 850 ml Aethanol, werden in Anwesenheit von 30,0 g Raney-Nickel bei Normaldruck bis zur Aufnahme der theoretischen Menge Wasserstoff hydriert. Durch externe Kühlung sorgt man dafür, dass die Reaktionstemperatur zwischen 15 und 20° C liegt. Nach Abtrennung des Raney-Nickels werden 90% des Aethanols abdestilliert, das Konzentrat in 500 ml Hexan aufgenommen, die Hexan-Phase mit Wasser, gesättigter Sodalösung und gesättigter Kochsalzlösung gewaschen, getrocknet und eingeengt. Die Destillation des so erhaltenen Rohproduktes (116,7 g) über eine 20 cm-Widmer-Kolonne ergibt 79,9 g (72%) olfaktorisch gutes 6-(Z)- und 6-(E)-Dihydrofarnesal (∼2:3) vom Kp_{0.05} 98 - 99 C.

Massenspektrum von 6-(Z)-2(3)-Dihydrofarnesal. 222(M⁺;0,2), 179(7), 161(4), 123(9), 109(13), 93(8), 81(14), 69(100), 55(11), 41(44).

Massenspektrum von 6-(E)-2(3)-Dihydrofarnesal. 222(M⁺;0,4), 179(7), 161(6), 123(13), 109(18), 93(9), 81(13), 69(100), 55(15), 41(46).

### Beispiel 2

### Parfümerie-Akkord blumiger Richtung mit einem Gehalt an I

| | Gewichtsteile | |
|---|---|---|
| | (1) | (2) |
| cis-3-Hexenol | 1 | 1 |
| cis-3-Hexenylacetat | 1 | 1 |
| Zitronenöl, italienisch | 10 | 10 |
| Linalool | 200 | 200 |
| Nerolidol | 100 | 100 |
| Benzylacetat | 50 | 50 |
| Methylbenzoat | 20 | 20 |
| Zimtalkohol | 40 | 40 |
| Cinnamylacetat | 50 | 50 |
| Nerol | 70 | 70 |
| Geraniol | 20 | 20 |
| Geranylacetat | 20 | 20 |
| β-Ionon | 5 | 5 |
| Eugenol | 6 | 6 |
| cis-3-Hexenylbenzoat | 15 | 14 |
| cis-3-Hexenylsalicylat | 30 | 30 |
| Methyljasmonat | 50 | 50 |
| Benzylbenzoat | 100 | 100 |
| ω-Pentadecanolid | 50 | 50 |
| 2(3)-Dihydrofarnesal (Beispiel 1) | -- | 100 |
| Dipropylenglycol | 162 | 62 |
| Total | 1000 | 1000 |

Der obige Parfümerie-Akkord (1) ist durch aromatisch-blumige Aspekte geprägt und erinnert zum Beispiel an Ylang-Ylang, Brunfelsia-Arten und nachtaktive Nicotiana-Arten. Der Zusatz von 100 Teilen I verleiht diesem Akkord die sehr geschätzte frisch-blumige Note, die sehr natürlich wirkt und an feuchte Blütenblätter erinnert. Diese geschätzte Note kann andererseits durch den äquivalenten Zusatz von beispielsweise Farnesol überhaupt nicht erzielt werden.

### Beispiel 3

### Parfümerie-Akkord blumiger Richtung mit teeartigen, würzigen und krautigen Aspekten

| | Gewichtsteile | |
|---|---|---|
| | (1) | (2) |
| Linalool | 200 | 200 |
| Bergamotteöl | 100 | 100 |
| Phenylethylalkohol | 100 | 100 |
| Citronellol | 80 | 80 |
| Geranylaceton | 50 | 50 |
| cis-3-Hexenylbenzoat | 50 | 50 |
| Anethol | 15 | 15 |
| Anisaldehyd | 5 | 5 |
| Methyljasmonat | 30 | 30 |
| Methyldihydrojasmonat | 40 | 40 |
| α-Terpineol | 30 | 30 |
| Geraniol | 20 | 20 |
| β-Ionon | 15 | 15 |
| Dihydro-ß-ionon | 15 | 15 |
| Eugenol | 5 | 5 |
| Phenylacetaldehyd | 5 | 5 |
| Citral | 5 | 5 |
| 2(3)-Dihydrofarnesal (Beispiel 1) | -- | 60 |
| Dipropylenglycol | 235 | 175 |
| | 1000 | 1000 |

Der obige, im Grundcharakter blumige Parfümerie-Akkord (1) ist durch zusätzliche teeartige, würzige und krautige Aspekte geprägt. Der Zusatz von 60 Teilen I bewirkt eine deutliche Verbesserung dieses Akkords, indem nun die teeartigen und krautigen Noten in optimaler Weise mit der blumigen Grundnote verbunden werden. Der so erhaltene Akkord 2 wirkt bedeutend harmonischer, besitzt eine wesentlich stärkere Ausstrahlung und zeichnet sich durch eine zusätzliche, sehr angenehme frischblumige Note aus. Der Akkord kann auch zur Modifizierung von Aromen verwendet werden.

Eine solche Verbesserung des Akkords kann durch den Zusatz von beispielsweise Farnesol in keiner Weise erzielt werden.

## Patentansprüche

1. Verwendung von 2(3)-Dihydrofarnesal der Formel 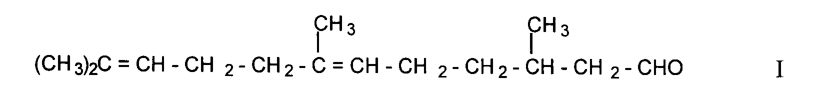 als Riech- und/oder Geschmackstoff.

2. Verwendung von 6-(E)- 2(3)-Dihydrofarnesal als Riech- und/oder Geschmackstoff.

3. Verwendung von 6-(Z)-2(3)-Dihydrofarnesal als Riech- und/oder Geschmackstoff.

4. Riech- und/oder Geschmackstoffkomposition, gekennzeichnet durch einen Gehalt an 2(3)-Dihydrofarnesal.

5. Riech- und/oder Geschmackstoffkomposition, gekennzeichnet durch einen Gehalt an 6-(E)-2(3)-Dihydrofarnesal.

6. Riech- und/oder Geschmackstoffkomposition, gekennzeichnet durch einen Gehalt an 6-(Z)-2(3)-Dihydrofarnesal.

7. Das 6-(E)-Isomere von 2(3)-Dihydrofarnesal als Riech- und/oder Geschmackstoff.

8. Das 6-(Z)-Isomere von 2(3)-Dihydrofarnesal als Riech- und/oder Geschmackstoff.

## Claims

1. The use of 2(3)-dihydrofarnesal of the formula 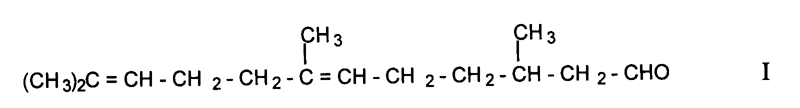 as an odorant and/or flavorant.

2. The use of 6-(E)-2(3)-dihydrofarnesal as an odorant and/or flavorant.

3. The use of 6-(Z)-2(3)-dihydrofarnesal as an odorant and/or flavorant.

4. An odorant and/or flavorant composition, characterized in that it contains 2(3)-dihydrofarnesal.

5. An odorant and/or flavorant composition, characterized in that it contains 6-(E)-2(3)-dihydrofarnesal.

6. An odorant and/or flavorant composition, characterized in that it contains 6-(Z)-2(3)-dihydrofarnesal.

7. The 6-(E) isomer of 2(3)-dihydrofarnesal as an odorant and/or flavorant.

8. The 6-(Z) isomer of 2(3)-dihydrofarnesal as an odorant and/or flavorant.

## Revendications

1. Utilisation de 2(3)-dihydrofarnésal de formule (I) 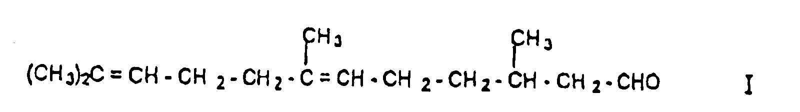 comme parfum et/ou comme matière donnant du goût.

2. Utilisation de 6-(E)-2(3)-dihydrofarnésal comme parfum et/ou comme matière donnant du goût.

3. Utilisation de 6-(Z)-2(3)-dihydrofarnésal comme parfum et/ou comme matière donnant du goût

4. Composition de parfum et/ou de matière donnant du goût, caractérisée en ce qu'elle contient du 2(3)-dihydrofarnésal.

5. Composition de parfum et/ou de matière donnant du goût, caractérisée en ce qu'elle contient du 6-(E)-2(3)-dihydrofarnésal.

6. Composition de parfum et/ou de matière donnant du goût, caractérisée en ce qu'elle contient du 6-(Z)-2(3)-dihydrofarnésal.

7. Isomère 6-(E)- de 2(3)-dihydrofarnésal comme parfum et/ou comme matière donnant du goût.

8. Isomère 6-(Z)- de 2(3)-dihydrofarnésal comme parfum et/ou comme matière donnant du goût.
